# EUROPEAN PATENT APPLICATION

(11) **EP 2 353 527 A2**
(43) Date of publication of application: **10.08.2011**
(21) Application number: 11250132.5
(22) Date of filing: 04.02.2011
(51) Int. Cl.: A61B 17/34

(54) **Port fixation with varying thread diameter**

(30) Priority: 05.02.2010 US 301783 P; 10.01.2011 US 987355
(71) Applicant: Tyco Healthcare Group LP, New Haven, CT 06511 (US)
(72) Inventor: Hammond, Richard, Northford, CT 06472 (US)
(74) Representative: Soames, Candida Jane

(57) **Abstract**

A surgical portal device for use in an endoscopic procedure includes an elongated body portion dimensioned for insertion through tissue. The body portion includes an outer wall defining a longitudinal axis and having a proximal end, a distal end, and a longitudinal lumen configured to allow a surgical instrument to pass therethrough. A plurality of threaded fixation segments is disposed on an exterior surface of the outer wall of the body portion. The threaded fixation segments are dimensioned and configured to engage tissue surrounding an opening to secure the elongated body at a predefined location within the tissue. The threaded fixation segments include a proximal threaded fixation segment having an average first thread diameter and a distal threaded fixation segment having an average second thread diameter less than the average first thread diameter. In one embodiment, each of the threaded fixation segments have different average thread diameters. The average thread diameters of the threaded fixation segments may gradually increase from a distal-most threaded fixation segment to a proximal- most threaded fixation segment.

## Description

### CROSS REFERENCE TO RELATED APPLICATION

The present application claims the benefit of and priority to U.S. Provisional Application Serial No. 61/301,783 filed on February 5, 2010, the entire contents of which are incorporated herein by reference.

### BACKGROUND

### Technical Field

The present disclosure relates to surgical ports. More particularly, the present disclosure relates to surgical access ports having port fixation components to secure the surgical access port relative to tissue of a patient.

### Background of Related Art

Surgical ports, such as introducers, trocars, and cannulas, permit the introduction of a variety of surgical instruments into a body cavity or opening within a patient. In procedures, such as endoscopic, laparoscopic or arthroscopic surgeries, a passage is created through tissue to access an underlying surgical site in the body. A port or cannula is positioned within the passage. Surgical instruments are introduced within the cannula to perform a surgical procedure. It may be advantageous to provide a portal device that can be removably placed within an incision or body opening of a patient to fix the access device therein.

### SUMMARY

Accordingly, a surgical portal device for use in an endoscopic procedure includes an elongated body portion dimensioned for insertion through tissue. The body portion includes an outer wall defining a longitudinal axis and having a proximal end, a distal end, and a longitudinal lumen configured to allow a surgical instrument to pass therethrough. A plurality of threaded fixation segments is disposed on an exterior surface of the outer wall of the body portion. The threaded fixation segments are dimensioned and configured to engage tissue surrounding an opening to secure the body portion at a predefined location within the tissue. The threaded fixation segments include a proximal threaded fixation segment having an average first thread diameter and a distal threaded fixation segment having an average second thread diameter less than the average first thread diameter. In one embodiment, each of the threaded fixation segments has different average thread diameters. The average thread diameters of the threaded fixation segments may gradually increase from a distal-most threaded fixation segment to a proximal- most threaded fixation segment.

In another embodiment, the threaded fixation segments include a first set of threaded fixation segments and a second set of threaded fixation segments distal of the first set. At least one of the threaded fixation segments of the first set defines the average first thread diameter and at least one of the threaded fixation segments of the second set defines the average second thread diameter. Each of the threaded fixation segments of the first set may define substantially equal average thread diameters and wherein each of the thread diameters.

The adjacent threaded fixation segments may be coterminous to define a single continuous thread along the exterior surface of the outer wall of the body portion. In the alternative, adjacent threaded fixation segments may be spaced to define a plurality of individual threaded fixation segments along the exterior surface of the outer wall of the body portion.

A distal-most threaded fixation segment may end abruptly without any transition toward the body portion and have a distal surface defining an angle ranging from about 60 degrees to about 90 degrees relative to the longitudinal axis of the body portion. This arrangement will facilitate immediate securement of the elongated body relative to tissue upon initial advancement within the tissue. In embodiments, the threaded fixation segment may define a length of extension "e" from the body portion and a thickness or width "w", and wherein the ratio of "e" to "w" is at least about 2:1.

An instrument seal may be mounted relative to the body portion. The instrument seal has inner surfaces defining a seal passage dimensioned for reception of the surgical instrument in substantial sealed relation therewith. A zero closure valve may be mounted relative to the body portion. The zero closure valve is adapted to close in the absence of the surgical instrument to substantially close the longitudinal lumen of the body portion.

The outer wall of the body portion may be substantially cylindrical.

A method of performing an endoscopic surgical procedure is disclosed. The method includes the steps of:
an elongated body dimensioned for insertion through a tissue tract, the body including an outer wall defining a longitudinal axis and having a proximal end, a distal end, and a longitudinal lumen configured to allow a surgical instrument to pass therethrough; and
a plurality of threaded fixation segments disposed on an exterior surface of the outer wall of the body, the threaded fixation segments including a proximal threaded fixation segment having an average first thread diameter and a distal threaded fixation segment having an average second thread diameter less than the average first thread diameter;
introducing the elongated body of the portal device within the tissue tract; based on a user determination of the size of the tissue tract, advancing the elongated body within the tissue to a position where one of the proximal threaded fixation segment and distal threaded fixation segment engages the tissue portions defining the tissue tract ; introducing a surgical instrument through the lumen of the elongated body; and
performing a surgical task with the surgical instrument.

### BRIEF DESCRIPTION OF THE DRAWINGS

Embodiments of the presently disclosed surgical portal device are described herein with reference to the accompanying drawings, wherein:

FIG. 1 is a perspective view of a surgical portal device according to an embodiment of the present disclosure;

FIG. 2 is a longitudinal cross-sectional view of the surgical portal device of FIG. 1 illustrated partially within tissue;

FIG. 3 is an axial view of the distal end of the surgical portal device;

FIG. 4 is a perspective view of a surgical portal device according to another embodiment of the present disclosure;

FIG. 5 is a longitudinal cross-sectional view of the surgical portal device of FIG. 4 illustrated partially within tissue; and

FIG. 6 is a flow chart illustrating a surgical method incorporating the surgical portal device.

Other features of the present disclosure will become apparent from the following detailed description, taken in conjunction with the accompanying drawings, which illustrate, by way of example, various principles of the present disclosure.

### DETAILED DESCRIPTION OF THE EMBODIMENTS

Particular embodiments of the present disclosure will be described herein with reference to the accompanying drawings. As shown in the drawings and as described throughout the following description, and as is traditional when referring to relative positioning on an object, the term "proximal" refers to the portion of the apparatus that is closer to the user and the term "distal" refers to the portion of the apparatus that is farther from the user. In the following description, well-known functions or constructions are not described in detail to avoid obscuring the present disclosure in unnecessary detail.

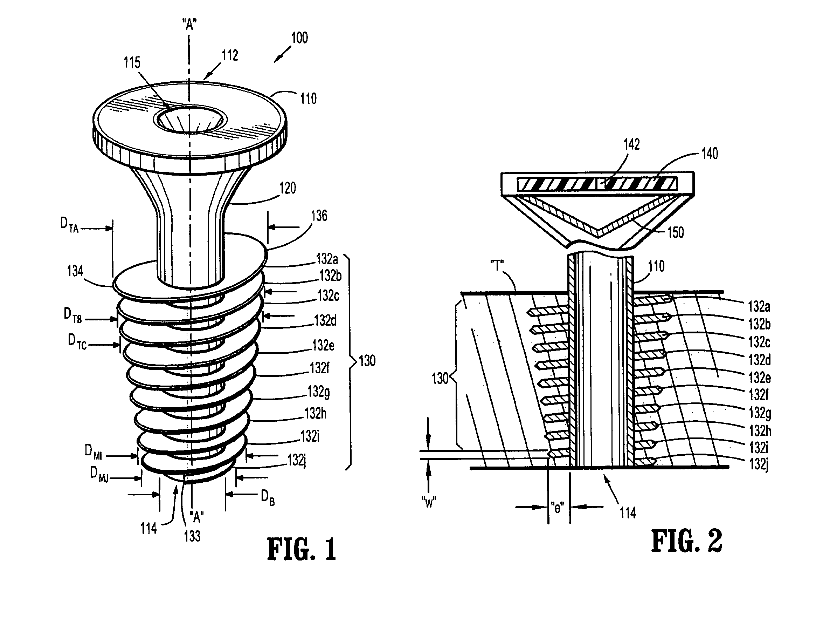

With reference to FIGS. 1-3, a surgical portal device 100 in accordance with embodiments of the present disclosure is shown. The surgical portal device 100 includes a body portion 110 and a threaded fixation portion 130. The body portion 110 includes a proximal end 112, a distal end 114, and a cylindrical bore or lumen 115 extending therethrough and defining a longitudinal axis "A-A." The lumen 115 is dimensioned for reception of at least one surgical instrument (not shown), including, but not limited to, clip appliers, graspers, dissectors, retractors, suture anchor installation systems, staplers, laser probes, photographic devices, endoscopes and laparoscopes, tubes, and the like.

The threaded fixation portion 130 includes a plurality of threaded fixation segments 132a, 132b, etc. (collectively referred to as "threaded fixation segments 132"). As used herein, the term "threaded fixation segment" relates to an at least partial revolution of the threaded fixation portion 130 about the longitudinal axis "A-A". While the term "threaded fixation segments" is used to describe portions of the threaded fixation portion 130, "threaded fixation segments" is not intended to mean or suggest that the threaded fixation portion 130 is not a single continuous thread. Rather, the threaded fixation portion 130 may include a single continuous thread or a plurality of discontinuous threads. In one embodiment, the threaded fixation segments 132 are substantially helical in shape. The threaded fixation segments 132 are dimensioned to engage surrounding tissue defining an opening to fixate the body portion 110 relative to tissue in a manner where the body portion 110 will not become inadvertently dislodged or expelled from the tissue in response to underlying pressure associated with the surgical procedure, e.g. when exposed to a pressurized abdominal cavity in a laparoscopic procedure or irrigant or distending fluid or saline in an arthroscopic procedure. Moreover, the dimensioning of the threaded fixation segments 132 ensures adequate engagement with the tissue by providing a substantially increased surface area contacting the skin while presenting a narrow profile to enhance penetration through the tissue. The threaded fixation segments 132 also may facilitate advancement of the body portion 110 within tissue; however, this may not be the primary function of the threaded fixation segments 132.

The body portion 110 includes an outer surface 120 having a substantially constant outer diameter D_{B} along a majority of its length. Each threaded fixation segment 132 includes a thread dimension or diameter (e.g., D_{TA} is the thread diameter of threaded fixation segment 132a) defined as the distance between opposite lateral outer edges 134, 136 of the threaded fixation segment 132 (see FIG. 1). The term diameter is to include circular cross-sections of the threaded fixation segment 132 , elliptical cross-sections or any other geometric shapes, i.e., the term diameter is not to be interpreted as restricted to a thread having only a circular cross-section, but will include other geometries. In the embodiment illustrated in FIG. 1, the proximal-most threaded fixation segment is labeled 132a and the distal-most threaded fixation segment is labeled 132k. While 10 threaded fixation segments (i.e., 132a, 132b, 132c, 132d, 132e, 132f, 132g, 132h, 132i, and 132j) are illustrated, it is envisioned that body portion 110 includes more or fewer threaded fixation segments 132 therearound.

In the embodiment illustrated in FIGS. 1-3, the thread diameter gradually decreases from proximal-most threaded fixation segment 132a to distal-most threaded fixation segment 132j. That is, the thread diameter D_{TA} of proximal-most threaded fixation segment 132a is larger than the thread diameter D_{TB} of distally adjacent threaded fixation segment 132b, which is larger than the thread diameter of D_{TC} of distally adjacent threaded fixation segment 132c, etc. This gradual decrease in thread diameter continues towards the distal end 114 of body portion, where the thread diameter D_{TJ} of distal-most threaded fixation segment 130j is smaller than the thread diameter D_{TI} of proximally adjacent threaded fixation segment 130i. It is envisioned that the thread diameter D_{TA} of proximal-most threaded fixation segment 132a is between about 0.15 inches and about 0.25 inches. It is envisioned that the thread diameter D_{TJ} of distal-most threaded fixation segment 130j is between about 0.01 inches and about 0.10 inches.

As best depicted in FIG. 2, thre aded fixation segments 132 define a thickness or width "w" which is substantially less than the degree or length of extension "e" of the thread fixations segments 132 from the outer diameter of the body portion 110, particularly, adjacent the proximal-most threaded fixation segment 132a. In embodiments, the ration of the degree of extension "e" to the width "w" may range from about 2:1 to about 6:1. This relatively low profile provided by this dimensioning will 1) facilitate relatively deep penetration of the threaded fixation segments 132 within the tissue and 2) provide a substantially increased surface area of contact of the threaded fixation segment 132a with the tissue. The combined result produces a substantially secured relation of the body portion 110 relative to tissue.

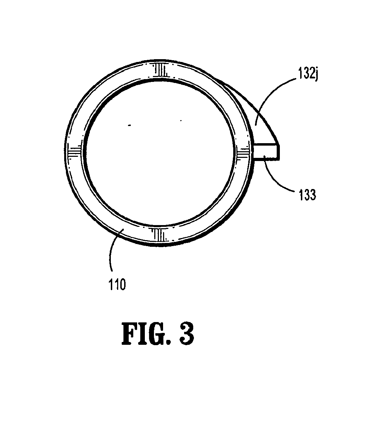

In the embodiment of FIGS. 1 -3, the distal-most threaded fixation segment 132j ends abruptly, i.e., without any transition or tapering toward the outer diameter of the body portion 110. For example, the distal most edge 133 of distal-most threaded fixation segment 132j is not flush with the outer surface of the body portion 110, but may extend at an angle to the longitudinal axis "A" of the body portion 110. Thus, upon initial entry of body portion within tissue, the distal-most threaded fixation segment 132j immediately functions to secure the body portion 110 to the tissue. In embodiments, the distal-most threaded fixation segment 132j extends at an angle of about 60 degrees to about 90 degrees relative to the longitudinal axis "A", and, particularly, may be orthogonal (or at 90 degrees) to the axis "A" as best depicted in FIG. 3.

Additionally, while the embodiment illustrated in FIGS. 1 -3 depicts a substantially linear change in thread diameter (i.e., the difference between adjacent threaded fixation segment diameters is substantially constant between the proximal-most threaded fixation segment 130a and the distal-most threaded fixation segment 130k), it is envisioned that the change in threaded fixation segment diameter along body portion 110 is non-linear (e.g., adjacent opposite lateral outer edges 134, 136 define a concave curve and/or a convex curve).

Surgical portal device 100 may further include an instrument seal 140 mounted adjacent proximal end 112. Instrument seal 140 may have inner surfaces defining a passage or aperture 142 for reception and passage of the surgical instrument. The inner surfaces establish a substantial fluid tight relation about the instrument to prevent or minimize the passage of fluids through elongated body portion 110. One preferred instrument seal is the seal disclosed in commonly assigned U.S. Patent No. 6,702,787 to Racenet, the entire contents of such disclosure being incorporated herein by reference. Surgical portal device 100 may further include a zero-closure valve 150 in the form of a duck-bill valve, trumpet valve or the like. Zero closure valve is adapted to open to permit passage of the surgical instrument and will close in the absence of the surgical instrument to substantially seal the longitudinal lumen 115 of the body portion 110.

Surgical portal device 100 may be used in conjunction with endoscopic surgical procedures such as laparoscopic procedures or arthroscopic procedures. In a laparoscopic surgical procedure, the abdominal cavity is insufflated with CO2 gas. The surgical portal device may be introduced through the abdominal wall with or without a surgical obturator as is known in the art. Depending on the procedure and size of the incision within the abdominal wall, the clinician may advance the surgical portal 100 to a desired location relative to the body wall to position a selected threaded fixation segment in engagement with the wall. Specifically, the selected threaded fixation segment will define a thread diameter which is suitable to secure the body portion 110 within the wall without being traumatic to the wall. In this regard, the different sized threaded fixation segments provide flexibility to the clinician. Instrument seal 140 will provide a substantial seal about the surgical instrument to minimize passage of gases through the body portion 110. In an arthroscopic procedure, the clinician can make a determination based on the size of the tissue tract as to the degree of advancement of the body portion and appropriate location of the desired thread size and segment relative to the tissue defining the tract. Instrument seal 140 will provide a substantial seal about a surgical instrument substantially minimizing the passage of fluids, saline, irrigant or the like to pass through the body portion 110.

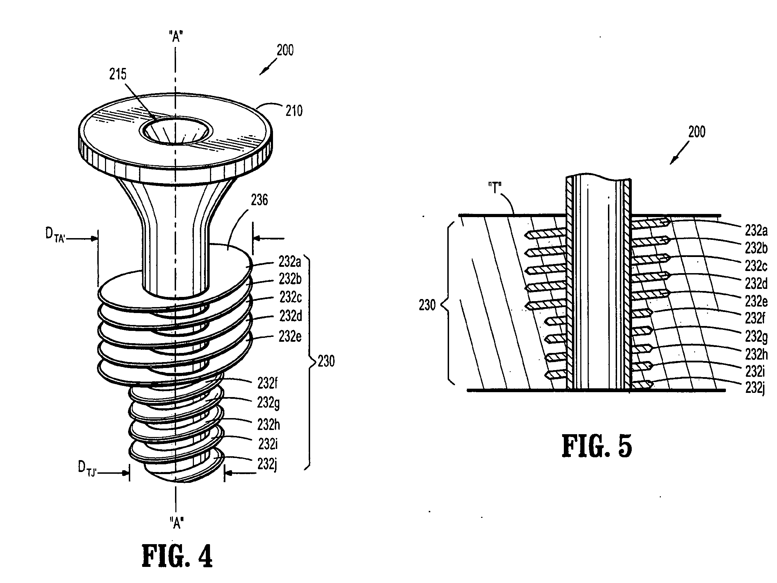

With reference to FIGS. 4 and 5, another embodiment of surgical portal device 200 is shown. Surgical portal device 200 of this embodiment includes threaded fixation portion 230. Similarly to the embodiment of FIGS. 1-3, threaded fixation portion 230 includes a plurality of threaded fixation segments 232a, 232b, etc. (collectively referred to as "threaded fixation segment 232" or "threaded fixation segments 232"). While the term "threaded fixation segments" is used to describe portions of the threaded fixation portion 230, "threaded fixation segments" is not intended to mean or suggest that the threaded fixation portion 230 is not a single continuous thread. Rather, the threaded fixation portion 230 may include a single continuous thread or a plurality of discontinuous threads. For example, the threaded fixation portion 230 may include a first proximal thread (including threads 232a, 232b, 232c, 232d and 232e), which is separate from a second distal thread (including threads 232f, 232g, 232h, 232i and 232j).

With continued reference to FIGS. 4 and 5, each threaded fixation segment 232 includes a thread diameter (e.g., D_{TA'} is the thread diameter of threaded fixation segment 232a) defined as the distance between opposite lateral outer edges 234, 236 of threaded fixation segment 232a (see FIG. 4). In the embodiment illustrated in FIGS. 4 and 5, the proximal-most threaded fixation segment is labeled 232a and the distal-most threaded fixation segment is labeled 232j. While, ten threaded fixation segments (i.e., 232a, 232b, 232c, 232d, 232e, 232f, 232g, 232h, 232i and 232j) are illustrated, it is envisioned that body portion 210 includes more or fewer threaded fixation segments 232 therearound.

In the embodiment illustrated in FIGS. 4 and 5, the thread diameter of each of the first set of five proximal-most threaded fixation segments (i.e., 232a, 232b, 232c, 232d and 232e) is substantially equal to one another. And the thread diameter of each of the second set of five distal-most threaded fixation segments (i.e., 232f, 232g, 232h, 232i and 232j) is substantially equal to one another. It is envisioned that the thread diameter (D_{TA'}) of the first set of five proximal-most threaded fixation segments is between about 0.15 inches and about 0.25 inches. It is envisioned that the thread diameter (including D_{TJ'}) of the second set of five distal-most threaded fixation segments is between about 0.01 inches and about 0.10 inches.

Thus, in contrast to the embodiment illustrated in FIGS. 1-3, the transition between the proximal threaded fixation portion and the distal threaded fixation portion in the embodiment illustrated in FIGS. 4 and 5 is abrupt. However, while the embodiment illustrated in FIGS. 4 and 5 includes threaded fixation portion 230 having only two sizes of thread diameters (i.e., D_{TA'} and D_{TJ'}), it is envisioned that threaded fixation portion 230 includes at least one threaded fixation segment located between threads 232e and 232f which has a thread diameter that is sized between and thread diameters D_{TA'} and D_{TJ'}; it is envisioned that a threaded fixation segment is positioned proximally of threaded fixation segment 232a and has a thread diameter that is larger than thread diameter D_{TA'}; and it is envisioned that a threaded fixation segment is positioned distally of threaded fixation segment 232j and has a thread diameter that is smaller than thread diameter D_{TJ'}.

In accordance with the embodiments of the present disclosure, threaded fixation segments 132, 232 of surgical portal device 100, 200 assist in removably securing surgical portal device 100, 200 within tissue "T." It is envisioned that the relatively small distal threaded fixation segments of threaded fixation portions 130, 130' (having a relatively small average thread diameter) facilitate entry of surgical portal device 100 into an incision, as it may reduce the trauma to the surrounding tissue "T" (see FIGS. 2 and 5). It is also envisioned that the relatively large proximal threaded fixation segments of threaded fixation portions 130, 130' (having a relatively large average thread diameter) enhances fixation within tissue "T." That is, when body portion 110 is adjacent tissue "T," the large proximal threaded fixation segments have a greater surface area that contacts the tissue "T." This provides a greater resistance to a proximally-directed force, which helps maintain the relative longitudinal position of surgical portal device 100 with respect to adjacent tissue "T."

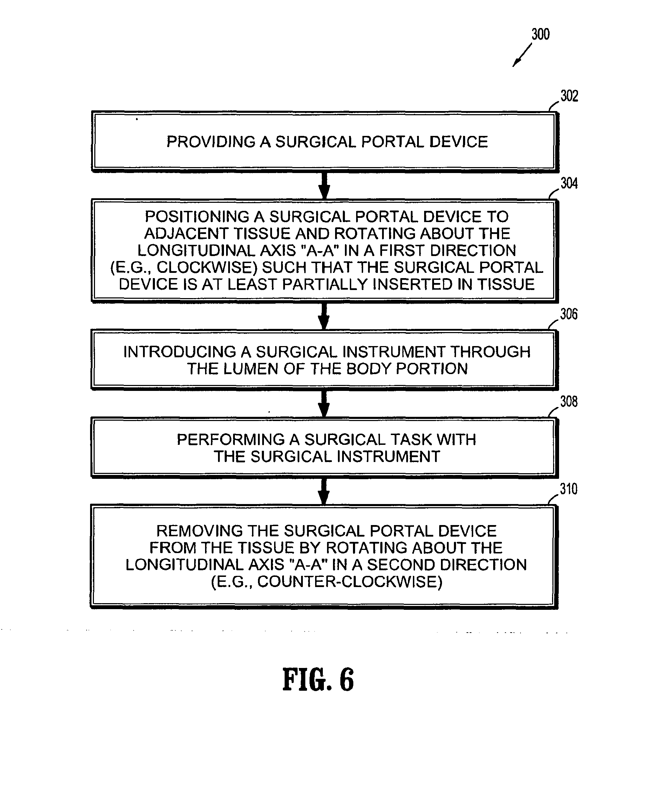

The present disclosure also relates to surgical methods utilizing the surgical portal device 100, 200. FIG. 6 is a flow chart illustrating one exemplary method 300 of use of the surgical portal device 100, 200. In one embodiment, the surgical portal device 100, 200 is used in conjunction with an endoscopic surgical procedure such as a laparoscopic surgical procedure of an arthroscopic surgical procedure. In accordance with the method 300, the surgical portal device 100, 200 including threaded fixation portion 130, 230 is provided (Step 302). The surgical portal device 100, 200 is positioned adjacent tissue and is rotated about the longitudinal axis "A-A" in a first direction (e.g., clockwise) such that the surgical portal device 100, 200 is at least partially inserted in a tissue tract within tissue (Step 304). The tissue tract may be a natural orifice of channel in the body or may include a tract created by the user during the procedure. This step may further include a determination by the user as to the relative size of the tissue tract within tissue. Based on this determination, the user will determine the degree of advancement of the body portion to position the desired size threaded fixation segment(s) 132 into engagement with the tissue defining the tissue tract. A surgical instrument is introduced through the lumen 115, 215 of the body portion 110, 210 (Step 306) followed by performance of a surgical task (e.g., endoscopic, laparoscopic, arthroscopic or the like) with the surgical instrument (Step 308). The surgical portal device 100, 200 may be removed from the tissue by being rotated about the longitudinal axis "A-A" in a second direction (e.g., counter-clockwise) (Step 310).

It will be understood that various modifications may be made to the embodiments of the presently disclosed portal device. Therefore, the above description should not be construed as limiting, but merely as exemplifications of embodiments. Those skilled in the art will envision other modifications within the scope and spirit of the present disclosure.
The invention may be described by reference to the following numbered paragraphs:-
1. A surgical portal device for use in an endoscopic procedure, comprising an elongated body dimensioned for insertion through tissue, the body including an outer wall defining a longitudinal axis and having a proximal end, a distal end, and a longitudinal lumen configured to allow a surgical instrument to pass therethrough; and a plurality of threaded fixation segments disposed on an exterior surface of the outer wall of the body portion, the threaded fixation segments dimensioned and configured to engage tissue surrounding an opening to secure the elongated body at a predefined location within the tissue, the threaded fixation segments including a proximal threaded fixation segment having an average first thread diameter and a distal threaded fixation segment having an average second thread diameter less than the average first thread diameter.
2. The surgical portal device of paragraph 1, wherein each of the threaded fixation segments has different average thread diameters.
3. The surgical portal device of paragraph 2, wherein the average thread diameters of the threaded fixation segments gradually increases from a distal-most threaded fixation segment to a proximal-most threaded fixation segment.
4. The surgical portal device of paragraph 1, wherein the threaded fixation segments include a first set of threaded fixation segments and a second set of threaded fixation segments distal of the first set, at least one of the threaded fixation segments of the first set defining the average first thread diameter and at least one of the threaded fixation segments of the second set defining the average second thread diameter.
5. The surgical portal device of paragraph 4, wherein each of the threaded fixation segments of the first set define substantially equal average thread diameters and wherein each of the threaded fixation segments of the second set define substantially equal average thread diameters
6. The surgical portal device of paragraph 1 wherein adjacent threaded fixation segments are coterminous to define a single continuous thread along the exterior surface of the outer wall of the body portion.
7. The surgical portal device of paragraph 1 wherein adjacent threaded fixation segments are spaced to define a plurality of individual threaded fixation segments along the exterior surface of the outer wall of the body portion.
8. The surgical portal device of paragraph 1 wherein a distal-most threaded fixation segment includes a distal surface defining an angle ranging from about 60 degrees to about 90 degrees relative to the longitudinal axis of the body portion.
9. The surgical portal device of paragraph 1 wherein the threaded fixation segments define a length of extension "e" from the body portion and a thickness or width "w", and wherein the ratio of "e" to "w" is at least about 2:1.
10. The surgical portal device of paragraph 1 including an instrument seal mounted relative to the body portion, the instrument seal having inner surfaces defining a seal passage dimensioned for reception of the surgical instrument in substantial sealed relation therewith.
11. The surgical portal device of paragraph 10 including a zero closure valve mounted relative to the body portion, the zero closure valve adapted to close in the absence of the surgical instrument to substantially close the longitudinal lumen of the body portion.
12. The surgical portal device of paragraph 1 wherein the outer wall of the body portion is substantially cylindrical.
13. A method of performing an endoscopic surgical procedure, comprising the steps of providing a surgical portal device including an elongated body dimensioned for insertion through a tissue tract, the body including an outer wall defining a longitudinal axis and having a proximal end, a distal end, and a longitudinal lumen configured to allow a surgical instrument to pass therethrough; and a plurality of threaded fixation segments disposed on an exterior surface of the outer wall of the body, the threaded fixation segments including a proximal threaded fixation segment having an average first thread diameter and a distal threaded fixation segment having an average second thread diameter less than the average first thread diameter, introducing the elongated body of the portal device within the tissue tract, based on a determination of the size of the tissue tract, advancing the elongated body within the tissue to a position where one of the proximal threaded fixation segment and distal threaded fixation segment engages the tissue portions defining the tissue tract, introducing a surgical instrument through the lumen of the elongated body; and performing a surgical task with the surgical instrument.
14. The method of paragraph 13, wherein the step of inserting at least a portion of the surgical portal device within tissue includes rotating the surgical portal device about the longitudinal axis in a first direction.
15. The method of paragraph 14, further comprising the step of removing the surgical portal device from tissue.
16. The method of paragraph 15, wherein the step of inserting at least a portion of the surgical portal device within tissue includes rotating the surgical portal device about the longitudinal axis in a first direction and wherein the step of removing the surgical portal device from tissue includes rotating the surgical portal device about the longitudinal axis in a second direction.

## Claims

1. A surgical portal device for use in an endoscopic procedure, comprising:
an elongated body dimensioned for insertion through tissue, the body including an outer wall defining a longitudinal axis and having a proximal end, a distal end, and a longitudinal lumen configured to allow a surgical instrument to pass therethrough; and
a plurality of threaded fixation segments disposed on an exterior surface of the outer wall of the body portion, the threaded fixation segments dimensioned and configured to engage tissue surrounding an opening to secure the elongated body at a predefined location within the tissue, the threaded fixation segments including a proximal threaded fixation segment having an average first thread diameter and a distal threaded fixation segment having an average second thread diameter less than the average first thread diameter.

2. The surgical portal device of claim 1, wherein each of the threaded fixation segments has different average thread diameters, preferably wherein the average thread diameters of the threaded fixation segments gradually increases from a distal-most threaded fixation segment to a proximal-most threaded fixation segment.

3. The surgical portal device of claim 1 or claim 2, wherein the threaded fixation segments include a first set of threaded fixation segments and a second set of threaded fixation segments distal of the first set, at least one of the threaded fixation segments of the first set defining the average first thread diameter and at least one of the threaded fixation segments of the second set defining the average second thread diameter, preferably wherein each of the threaded fixation segments of the first set define substantially equal average thread diameters and wherein each of the threaded fixation segments of the second set define substantially equal average thread diameters

4. The surgical portal device of any preceding claim wherein adjacent threaded fixation segments are coterminous to define a single continuous thread along the exterior surface of the outer wall of the body portion.

5. The surgical portal device of any preceding claim wherein adjacent threaded fixation segments are spaced to define a plurality of individual threaded fixation segments along the exterior surface of the outer wall of the body portion.

6. The surgical portal device of any preceding claim wherein a distal-most threaded fixation segment includes a distal surface defining an angle ranging from about 60 degrees to about 90 degrees relative to the longitudinal axis of the body portion.

7. The surgical portal device of any preceding claim wherein the threaded fixation segments define a length of extension "e" from the body portion and a thickness or width "w", and wherein the ratio of "e" to "w" is at least about 2:1.

8. The surgical portal device of any preceding claim including an instrument seal mounted relative to the body portion, the instrument seal having inner surfaces defining a seal passage dimensioned for reception of the surgical instrument in substantial sealed relation therewith.

9. The surgical portal device of claim 8 including a zero closure valve mounted relative to the body portion, the zero closure valve adapted to close in the absence of the surgical instrument to substantially close the longitudinal lumen of the body portion.

10. The surgical portal device of any preceding claim wherein the outer wall of the body portion is substantially cylindrical.

11. A surgical portal device according to any preceding claim including:
an elongated body dimensioned for insertion through a tissue tract, the body including an outer wall defining a longitudinal axis and having a proximal end, a distal end, and a longitudinal lumen configured to allow a surgical instrument to pass therethrough; and
a plurality of threaded fixation segments disposed on an exterior surface of the outer wall of the body, the threaded fixation segments including a proximal threaded fixation segment having an average first thread diameter and a distal threaded fixation segment having an average second thread diameter less than the average first thread diameter, for use in a method of performing an endoscopic surgical procedure, wherein the method comprises the step of:.
introducing the elongated body of the portal device within the tissue tract;
based on a determination of the size of the tissue tract, advancing the elongated body within the tissue to a position where one of the proximal threaded fixation segment and distal threaded fixation segment engages the tissue portions defining the tissue tract ;
introducing a surgical instrument through the lumen of the elongated body; and
performing a surgical task with the surgical instrument.

12. A surgical device for use according to the method of claim 11, wherein the step of inserting at least a portion of the surgical portal device within tissue includes rotating the surgical portal device about the longitudinal axis in a first direction.

13. A surgical device for use according to the method of claim 11 or claim 12, further comprising the step of removing the surgical portal device from tissue.

14. A surgical device for use according to the method of any of claims 11 to 13, wherein the step of inserting at least a portion of the surgical portal device within tissue includes rotating the surgical portal device about the longitudinal axis in a first direction and wherein the step of removing the surgical portal device from tissue includes rotating the surgical portal device about the longitudinal axis in a second direction.
